# EUROPÄISCHE PATENTSCHRIFT

(11) **EP 2 656 821 B1**
(45) Veröffentlichungstag und Bekanntmachung des Hinweises auf die Patenterteilung: **08.06.2016**
(21) Anmeldenummer: 13002152.0
(22) Anmeldetag: 24.04.2013
(51) Int. Cl.: A61F 5/56

(54) **Schnarchhemmende Mundspange**
Snore-inhibiting mouth brace
Appareil buccal empêchant le ronflement

(30) Priorität: 24.04.2012 CH 5592012
(43) Veröffentlichungstag der Anmeldung: 30.10.2013
(73) Patentinhaber: Scheuermann, Walter, 8262 Ramsen (CH); Scheuermann, Ursula, 8262 Ramsen (CH)
(72) Erfinder: Scheuermann, Walter, 8262 Ramsen (CH); Scheuermann, Ursula, 8262 Ramsen (CH)
(74) Vertreter: Alder, Hans Rudi

(56) Entgegenhaltungen:
- DE-U1-202006 020 335
- US-A1- 2011 178 439
- US-A1- 2011 226 264

## Beschreibung

Die vorliegende Erfindung betrifft eine schnarchhemmende Mundspange gemäss Oberbegriff des Anspruchs 1.

Schnarchen zu hemmen oder ganz zu verhindern ist ein weit verbreitetes Anliegen in unserer Gesellschaft. Vorrichtungen, mit welchen das Schnarchen gehemmt oder ganz verhindert wird, sollen im Folgenden mit dem Adjektiv "schnarchhemmend" (inhibiting snoring) bezeichnet werden.

Eine solche schnarchhemmende Vorrichtung in Form einer Mundspange ist bspw. aus der WO2007/020197 bekannt. Diese umfasst ein drahtförmiges Element, insbesondere einen in einem Plastikschlauch angeordneten Federdraht, welches derart geformt ist, dass der weiche Gaumen und damit das Gaumensegel des Anwenders stabilisiert werden. Dies wird dadurch erreicht, dass rachenseitig ein mittlerer, resp. hinterer Bereich dieses drahtförmigen Elementes den weichen Gaumen bogenförmig hintergreift und sich die beiden vorderen Enden dieses drahtförmigen Elementes lippenseitig abstützen. Dadurch wird eine Klammerwirkung, d.h. eine Zugspannung zwischen dem weichen Gaumen und dem Lippenbereich erzeugt, welche verhindert, dass der weiche Gaumen beim entspannten Schlafen ins Flattern gerät und die unerwünschten Schnarchgeräusche erzeugt.

Es versteht sich, dass damit lediglich Schnarchgeräusche verhindert werden, welche vom weichen Gaumen, resp. dem Gaumensegel erzeugt werden. Schnarchgeräusche, welche von der Muskulatur der Rachenwand oder im Bereich des Zungengrunds resp. Kehldeckels erzeugt werden, können damit nicht verhindert werden.

Es ist deshalb die Aufgabe der vorliegenden Erfindung eine schnarchhemmende Mundspange zu schaffen, welche sowohl die vom weichen Gaumen erzeugten Schnarchgeräusche als auch die von der Rachenwand und dem Zungengrund erzeugten Schnarchgeräusche verhindert.

Erfindungsgemäss wird dies mit einer Mundspange mit den Merkmalen des Anspruchs 1 gelöst und insbesondere mit einer Mundspange, bei welcher zwei Spangenbügel über ein elastisch federndes Verbindungsstück miteinander verbunden sind, wobei jeder der Spangenbügel ein abgewinkeltes Spangenende, einen Spangenbogen und mindestens eine Gaumenabstützung umfasst und wobei jeder der Spangenbügel derart geformt und dimensioniert ist, dass dieser im eingesetzten Zustand rachenseitig mit der Gaumenabstützung den weichen Gaumen eines Anwenders hintergreift, um diesen Gaumen zu stabilisieren, und lippenseitig mit dem Spangenende im Lippenbereich des Anwenders derart anliegt, dass der Spangenbogen zwischen dem weichen Gaumen und dem Lippenbereich des Anwenders eingespannt ist, wobei das elastisch federnde Verbindungsstück zwischen den beiden Spangenbügeln eine Rachenwandabstützung und eine Zungengrundabstützung aufweist. Dieses Verbindungsstück ist derart geformt und dimensioniert, dass im eingesetzten Zustand die Rachenwandabstützung an der Rachenwand des Anwenders anliegt und die Zungengrundabstützung am Zungengrund des Anwenders anliegt, um die Rachenwand und den Zungengrund des Anwenders zu stabilisieren.

In einer bevorzugten Ausführungsform der erfindungsgemässen Mundspange sind die beiden Spangenbügel im Bereich zwischen der Gaumenabstützung und der Rachenwandabstützung über eine elastische Verbindung miteinander verbunden.

Um diese Nachteile zu überwinden wurde bspw. mit der US2011/0226264 eine Vorrichtung vorgeschlagen, welche Rückstellelemente in unterschiedlicher Form und Gestaltung aufweist. Diese Rückstellelemente sind ein- und auffaltbar und werden im eingefalteten Zustand über die Mundhöhle oder die Nasenhöhle hinter den weichen Gaumen und/oder hinter der Zunge platziert bevor sie dort aufgefaltet, resp. aufgeweitet werden, um mit einer geringen Kraft auf diese Gewebe zu wirken. Es versteht sich, dass eine derartige Vorrichtung eine aufwändige Fertigung erfordert und vom Anwender nur umständlich einsetzbar und reinigbar ist.

Eine in analoger Weise funktionierende Vorrichtung von denselben Erfindern wird in der US2011/0178439 beschrieben. Diese weist ein Rückstellelement auf, welches an einer Gaumenspange befestigt ist und als aufweitbares Element, insbesondere in Form eines rohrförmigen oder gitterartigen Elementes, insbesondere aus einem Werkstoff mit Formgedächtnis, in Form eines Drahtgeflechtes oder Drahtes mit Formgedächtnis, in Form eines aufweitbaren Stents oder in Form eines mit einem Magnet auffaltbaren Gitterwerks. Leider weisen all diese Vorrichtungen dieselben Nachteile der obigen Vorrichtung auf, d.h. erfordern ein aufwändiges Fertigungsverfahren und lassen sich im Gebrauch nicht oder nur umständlich reinigen, was aus hygienischen Gründen für wiederverwendbare Produkte im medizinischen Bereich unabdingbar wäre.

Es ist deshalb die Aufgabe der vorliegenden Erfindung, eine schnarchhemmende Mundspange zu schaffen, welche sowohl die vom weichen Gaumen erzeugten Schnarchgeräusche als auch die von der Rachenwand und dem Zungengrund erzeugten Schnarchgeräusche verhindert.

Erfindungsgemäss wird dies mit einer Mundspange mit den Merkmalen des Anspruchs 1 gelöst und insbesondere mit einer Mundspange, bei welcher zwei Spangenbügel über ein elastisch federndes Verbindungsstück miteinander verbunden sind, wobei jeder der Spangenbügel ein abgewinkeltes Spangenende, einen Spangenbogen und mindestens eine Gaumenabstützung umfasst und wobei jeder der Spangenbügel derart geformt und dimensioniert ist, dass dieser im eingesetzten Zustand rachenseitig mit der Gaumenabstützung den weichen Gaumen eines Anwenders hintergreift, um diesen Gaumen zu stabilisieren, und lippenseitig mit dem Spangenende im Lippenbereich des Anwenders derart anliegt, dass der Spangenbogen zwischen dem weichen Gaumen und dem Lippenbereich des Anwenders eingespannt ist, wobei das elastisch federnde Verbindungsstück zwischen den beiden Spangenbügeln eine Rachenwandabstützung und eine Zungengrundabstützung aufweist. Dieses Verbindungsstück ist derart geformt und dimensioniert, dass im eingesetzten Zustand die Rachenwandabstützung an der Rachenwand des Anwenders anliegt und die Zungengrundabstützung am Zungengrund des Anwenders anliegt, um die Rachenwand und den Zungengrund des Anwenders zu stabilisieren.

In einer bevorzugten Ausführungsform der erfindungsgemässen Mundspange sind die beiden Spangenbügel im Bereich zwischen der Gaumenabstützung und der Rachenwandabstützung über eine elastische Verbindung miteinander verbunden.

In einer bevorzugten Weiterbildung der erfindungsgemässen Mundspange weist diese mindestens teilweise einen elastisch verformbaren Draht auf. Damit lässt sich die Mundspange in einfacher Weise an die Form der Mundhöhle und des Rachenraums des Anwenders anpassen. Darüber hinaus ist dieser Draht wegen des Tragekomforts mit einem Kunststoffmantel umgeben, vorzugsweise aus einem weichelastischen Material. Es versteht sich, dass der Fachmann geeignete Werkstoffe für den elastisch verformbaren Draht auswählt und insbesondere physiologisch unbedenkliche Werkstoffe, wie sie auch aus der Medizintechnik bekannt sind, verwendet.

Es versteht sich, dass die erfindungsgemässe Mundspange einstückig oder mehrteilig aufgebaut sein kann. Insbesondere lässt sich diese Mundspange manuell oder maschinell anpassen, d.h. kann die Mundhöhle und der Rächenraum des Anwenders maschinell ausgemessen werden, um die Mundspange automatisch zu verformen.

In einer privilegierten Ausführungsform der vorliegenden Erfindung ist zwischen dem Draht und dem Kunststoffmantel ein nach der Formgebung eingebrachtes Kunststoffmaterial vorgesehen. Dieses nach der Formgebung eingebrachte Kunststoffmaterial ist ein fliessfähiger Kunststoff, insbesondere ein Silikon, welcher nach dem Einfüllen rasch polymerisiert.

Die Vorteile dieser Mundspange sind dem Fachmann unmittelbar ersichtlich und insbesondere in der einfachen Herstellungs- und Anwendungsweise sowie in der überraschend effektiven Wirkungsweise zu sehen.

Im Folgenden soll die Erfindung an einem Ausführungsbeispiel und mit Hilfe der Figuren näher erläutert werden. Dabei zeigt:
- Fig. 1:: eine schematische Darstellung einer erfindungsgemässen Mundspange im eingesetzten Zustand;
- Fig. 2:: eine schematische Seitenansicht der erfindungsgemässen Mundspange;
- Fig. 3:: eine schematische räumliche Darstellung der erfindungsgemässen Mundspange;
- Fig. 4a-c: schematische Darstellungen der erfindungsgemässen Mundspange in unterschiedlichen Stellungen.

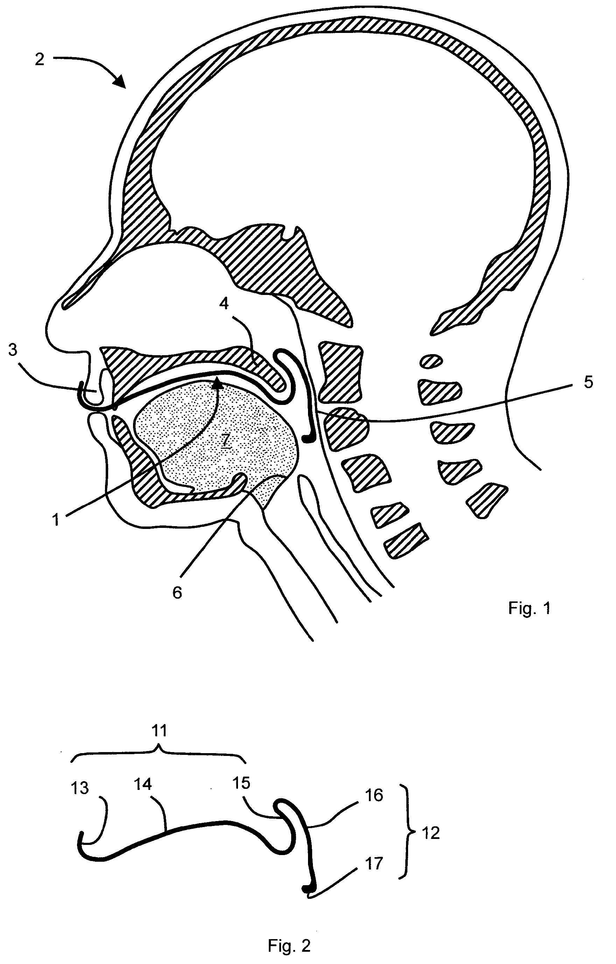

Die in Fig. 1 dargestellte Mundspange 1 liegt in der Mundhöhle eines Anwenders 2 und ist zwischen dem Lippenbereich 3 und dem weichen Gaumen 4 des Anwenders 2 eingespannt. Dabei hintergreift ein hinterer Teil dieser Mundspange 1 den weichen Gaumen 4 und erstreckt sich weiter entlang der Rachenwand 5 bis zum Zungengrund 6 der Zunge 7 des Anwenders 2. Erfindungsgemäss ist diese Mundspange derart geformt und dimensioniert, dass der weiche Gaumen 4, die Rachenwand 5 und der Zungengrund 6 des Anwenders 2 beim Atmen am freien Flattern oder Vibrieren gehindert wird, wie dies beim Schnarchen im Schlaf bei entspannter Muskulatur in störender Weise auftritt. Es versteht sich, dass die erfindungsgemässe Mundspange jedem Anwender individuell, d.h. der Form der Mundhöhle und des Rachenraums des Anwenders entsprechend angepasst werden muss, um die optimale Wirkung bei maximalem Tragekomfort zu erzielen. Es hat sich gezeigt, dass das Einsetzen einer solchen Mundspange nach einer kurzen Angewöhnungsphase vom Anwender ohne Brechreiz oder andere Beschwerden selber eingesetzt und getragen werden kann. In einer bevorzugten Ausführungsform ist die erfindungsgemässe Mundspange drahtförmig ausgebildet und ermöglicht eine ruhige und leichte Atmung. Die schnarchhemmende Wirkung, insbesondere das Verhindern von Atemaussetzern (Apnoe) führt bei den Anwendern zu einem tiefen und erholsamen Schlaf.

Fig. 2 zeigt eine Seitenansicht der erfindungsgemässen Mundspange 1 in schematischer Weise und lässt die einzelnen Funktionsbereiche derselben deutlich erkennen. Die erfindungsgemässe Mundspange umfasst zwei Spangenbügel 11, welche über ein elastisches Verbindungsstück 12 miteinander verbunden sind. Dabei weist jeder der Spangenbügel 11 ein Spangenende 13, einen Spangenbogen 14 und eine Gaumenabstützung 15 auf. Das elastische Verbindungsstück 12 ist im Bereich der Gaumenabstützung 15 mit den beiden Spangenbügeln 11 verbunden und umfasst mindestens eine Rachenwandabstützung 16 und eine Zungengrundabstützung 17. Es versteht sich, dass diese Funktionselemente 13 bis 17 einstückig oder aus einzeln gefertigten Elementen bestehen können. In einer bevorzugten Ausführungsform der erfindungsgemässen Mundspange 1 ist diese, wie bspw. in der zitierten WO2007/020197 offenbart, aus einem mit einem Kunststoffschlauch ummantelten Draht gefertigt.

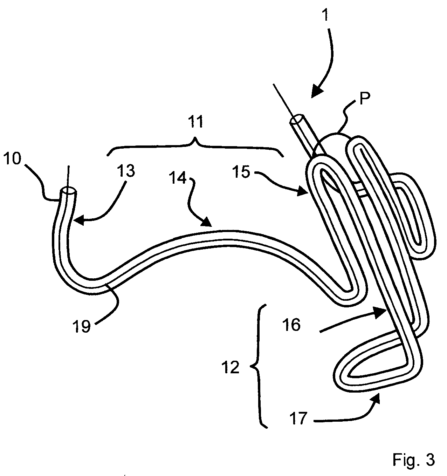

Eine bevorzugte Ausführungsform der erfindungsgemässen Mundspange 1 ist in Fig. 3 räumlich dargestellt und zeigt die Formgebung der beiden links- und rechtsseitigen Spangenbügel 11 mit ihren Funktionselementen 13, 14 und 15 sowie des elastischen Verbindungsstücks 12 mit seinen Funktionselementen 16 und 17 deutlich. Bei einer korrekt angepassten Mundspange 1 erzeugen das Spangenende 13 und die Gaumenabstützung 15 über den Spangenbogen 14 eine Klammerwirkung, welche den weichen Gaumen 4 stabilisiert. Das Gaumensegel des Anwenders kann sich zwischen den links- und rechtsseitigen Gaumenabstützungen 15 frei bewegen. Erfindungsgemäss sind die Gaumenabstützungen 15 mit einem elastischen Verbindungsstück 12 verbunden, welches eine Rachenwandabstützung 16 und eine Zungengrundabstützung 17 aufweist. Diese Rachenabstützung 16 und Zungengrundabstützung 17 liegen im eingesetzten Zustand an der Rachenwand resp. am Zungengrund der Zunge des Anwenders an und brauchen keinen Druck auf die Rachenwand oder den Zungengrund auszuüben. Dieses Verbindungsstück 12 hält lediglich das beim Schlafen erschlaffenden Gewebe des Anwenders zurück, um ein ungehindertes Atmen zu gewährleisten. Damit wird auch verhindert, dass der vorbeistreichende Luftstrom diese erschlafften Gewebepartien nicht oder nur wenig zum Flattern oder Vibrieren bringen kann. Es versteht sich, dass die erfindungsgemässe Mundspange 1 an die individuelle Mundhöhlen- und Rachenform angepasst werden muss. Dazu erweist sich die Verwendung eines mit Kunststoff ummantelten Drahtes 19 als besonders geeignet, insbesondere lässt sich durch eine geeignete Materialwahl die Elastizität und Formstabilität bestimmen. In einer bevorzugten Ausführungsform wird die Mundspange nach dem Anpassen mit einem silikonartigen Material verfüllt, d.h. wird der Zwischenraum zwischen dem geformten Draht 19 und dem Kunststoffmantel 10 aufgefüllt. Dies erlaubt in einem ersten Schritt eine einfache und damit präzise Verformung der Mundspange bei der Anpassung und in einem zweiten Schritt eine Erhöhung der Formstabilität nach der Anpassung. Es versteht sich, dass durch die präzise Anpassung der Mundspange der Tragekomfort erhöht werden kann und durch die erhöhte Formstabilität, die Mundspange auch eine verbesserte Langzeitstabilität aufweist.

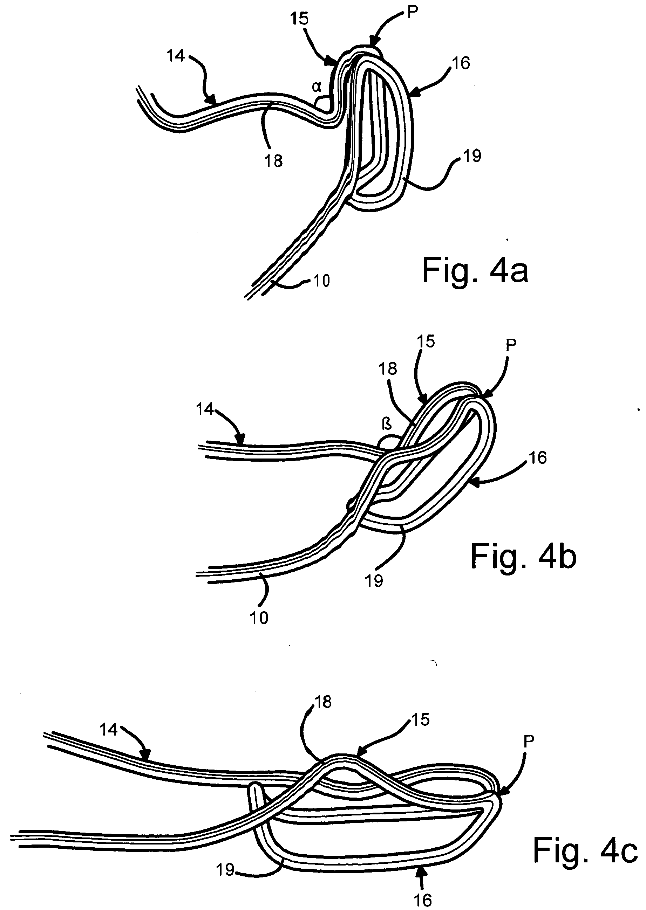

In einer bevorzugten Weiterbildung der erfindungsgemässen Mundspange sind die beiden Spangenbügel 11 im Bereich zwischen der Gaumenabstützung 15 und der Rachenwandabstützung 16 mit einer elastischen Verbindung P miteinander verbunden. Diese Verbindung P erlaubt dem Anwender ein vereinfachtes Einsetzen der fertig angepassten Mundspange, wie in den Fig. 4a-c näher erläutert werden soll.

Fig. 4a zeigt in schematischer Weise eine Mundspange 1 nach der Anpassung in ihrer gebrauchsbereiten Form mit einem in einem verfüllten Kunststoffmantel 10 liegenden, formgebenden Draht 19. Der Kunststoffschlauch oder -mantel 10 wird bei der vorliegenden Erfindung nach der abschliessenden Formgebung der Mundspange vorzugsweise mit einem selbsthärtenden und weichelastischen Kunststoffmaterial, insbesondere einem physiologisch unbedenklichen Silikon verfüllt. Als wesentliche Erleichterung hat sich die Verwendung einer Verbindung P zwischen der Gaumenabstützung 15 und der Rachenwandabstützung 16 erwiesen. In einer einfachen Ausführungsform wird dieses Verbindungselement P mittels eines ebenfalls eingegossenen Zusatzdrahtes 18 realisiert. Diese Figur 4a macht deutlich, dass vor dem Einsetzen der erfindungsgemässen Mundspange 1 die Spangenbogen 14 im Wesentlichen orthogonal, d.h. in einem Formwinkel α zu den Rachenwandabstützungen 16 liegen.

Verdreht man die Spangenenden 13 nach aussen und damit auch die jeweiligen Spangenbogen 14, kippen die Gaumenabstützungen 15, im Wesentlichen wegen der Verbindung P, nach hinten und neigen sich um einen Kippwinkel β > α nach hinten, d.h. rachenseitig zu einer von den Spangenbogen 14 aufgespannten Ebene. Dabei nehmen sie die Rachenwandabstützungen 16 mit, d.h. erzeugen ein Einklappen der Rachenwandabstützung 12, wie in Fig. 4b verdeutlicht.

Verdreht man die beiden Spangenenden 13, resp. die dazugehörenden Spangenbogen 14 in derselben Weise nach aussen weiter, legen sich die Gaumenabstützungen 15 rachenseitig in die Ebene der Spangenbogen 14 und lassen die Rachenwandabstützungen 16 einklappen, wie in Fig. 4c veranschaulicht. In dieser Stellung lässt sich die erfindungsgemässe Mundspange vom Anwender in einfacher Weise in den Rachenbereich einführen. Beim Zurückdrehen legt sich einerseits die Gaumenabstützung 15 hinter den weichen Gaumen des Anwenders und klappt andererseits gleichzeitig die Rachenwandabstützung 16 auf, um sich an die Rachenrückwand zu legen und den Zungengrund abzustützen. Damit weist die Mundspange 1 wieder ihre ursprüngliche an den Anwender individuell angepasste Form auf

Es versteht sich, dass die Zungengrundabstützung der erfindungsgemässen Mundspange zusätzlich verformt sein kann, insbesondere um einen am Zungengrund grossflächig anliegenden Verlauf der Zungengrundabstützung zu erzeugen. Die vorgängig beschriebenen Ausführungsformen sind nur beispielhaft aufgeführt. Der Fachmann wird insbesondere nach anderen geeigneten Materialien zur Fertigung einer solchen Mundspange suchen, insbesondere Kunststoffe die sich in einfacher Weise bearbeiten lassen, bspw. sich durch Wärmebehandlung verformen lassen, oder die geeignet sind eine erfindungsgemässe Mundspange, bspw. aufgrund eines maschinell ausgemessenen Rachenraums, automatisch zu fertigen, insbesondere durch Spritzgiessen.

### Bezugszeichenliste:

- 1: Mundspange
- 2: Anwender
- 3: Lippenbereich
- 4: weicher Gaumen
- 5: Rachenwand
- 6: Zungengrund
- 7: Zunge
- 10: Kunststoffmantel
- 11: Spangenbügel
- 12: Verbindungsstück
- 13: Spangenende
- 14: Spangenbogen
- 15: Gaumenabstützung
- 16: Rachenwandabstützung
- 17: Zungengrundabstützung
- 18: Zusatzdraht
- 19: Draht
- P: Verbindungselement
- α: Formwinkel
- β: Kippwinkel

## Patentansprüche

1. Schnarchhemmende Mundspange (1), mit zwei Spangenbügeln (11), welche über ein elastisch federndes Verbindungsstück (12) miteinander verbunden sind, wobei jeder der Spangenbügel (11) ein abgewinkeltes Spangenende (13), einen Spangenbogen (14) und mindestens eine Gaumenabstützung (15) umfasst, und jeder Spangenbügel (11) derart geformt und dimensioniert ist, dass dieser im eingesetzten Zustand rachenseitig mit der Gaumenabstützung (15) den weichen Gaumen eines Anwenders (2) hintergreift, um diesen Gaumen zu stabilisieren, und lippenseitig mit dem Spangenende (13) im Lippenbereich des Anwenders (2) derart anliegt, dass der Spangenbogen (14) zwischen dem weichen Gaumen und dem Lippenbereich des Anwenders (2) eingespannt ist, **dadurch gekennzeichnet, dass** das elastisch federnde Verbindungsstück (12) zwischen den beiden Spannbügeln (11) eine Rachenwandabstützung (16), die sich im eingesetzten Zustand entlang der Rachenwand (5) bis zum Zungengrund (6) der Zunge (7) erstreckt, und eine am zungengrundseitigen Ende der Rachenwandabstützung (16) liegende Zungengrundabstützung (17) aufweist, welche derart geformt und dimensioniert sind, dass im eingesetzten Zustand die Rachenwandabstützung (16) an der Rachenwand (5) des Anwenders (2) anliegt und die Zungengrundabstützung (17) am Zungengrund (6) des Anwenders (2) anliegt, um die Rachenwand (5) und den Zungengrund (6) des Anwenders (2) zu stabilisieren.

2. Mundspange nach Anspruch 1, **dadurch gekennzeichnet, dass** die beiden Spangenbügel (11) zwischen der Gaumenabstützung (15) und der Rachenwandabstützung (16) über ein elastisches Verbindungselement (P) miteinander verbunden sind.

3. Mundspange nach einem der Ansprüche 1 oder 2, **dadurch gekennzeichnet, dass** diese mindestens teilweise einen elastisch verformbaren Draht (19) aufweist.

4. Mundspange nach Anspruch 3, **dadurch gekennzeichnet, dass** dieser Draht (19) von einem Kunststoffmantel (10) umgeben ist.

5. Mundspange nach Anspruch 4, **dadurch gekennzeichnet, dass** der Kunststoffmantel (10) weichelastisch ist.

6. Mundspange nach Anspruch 1, **dadurch gekennzeichnet, dass** diese aus physiologisch verträglichem Material aufgebaut ist.

7. Mundspange nach Anspruch 1, **dadurch gekennzeichnet, dass** diese manuell oder maschinell verformbar ist.

8. Mundspange nach Anspruch 4, **dadurch gekennzeichnet, dass** diese zwischen dem Draht (19) und dem Kunststoffmantel (10) mit einem nach der Formgebung eingebrachten Kunststoffmaterial versehen ist.

9. Mundspange nach Anspruch 8, **dadurch gekennzeichnet, dass** das nach der Formgebung eingebrachte Kunststoffmaterial ein silikonartiger Kunststoff ist.

10. Mundspange nach Anspruch 1 oder 2, **dadurch gekennzeichnet, dass** diese einstückig ausgebildet ist.

## Claims

1. Snore preventing mouth brace (1), with two brace-bows (11), which are connected to one another by an elastically resilient connecting piece (12), whereby each of the brace-bows (11) comprises a bent brace-end (13), a brace-arc (14) and at least a palate-support (15), and each brace-bow (11) is shaped and dimensioned such that, when inserted, it is on its pharynx-side, reaching with its palate-support (15) behind the soft palate of a user (2) in order to stabilize this palate, and is on its lip side, closely fitting with its brace-end (13) on the lip of the user (2) such that the brace-bow (14) is clamped between the soft palate and the lip of the user (2),
**characterized in that**
the resilient connecting piece (12) between the two brace-bows (11) comprises a pharyngeal wall support (16), which extends along the pharyngeal wall (5) to the tongue base (6) of the tongue (7), and a tongue base support (17), which is lying at the radical, that is the pharynx-sided end of the pharyngeal wall support (16), which are shaped and dimensioned such that, when inserted, the pharyngeal wall support (16) abuts to the pharyngeal wall (5) of the user (2) and the tongue base support (17) abuts to the tongue base (6) of the user (2) in order to stabilize the pharyngeal wall (5) and the tongue base (6) of the user (2).

2. Mouth brace according to claim 1, **characterized in that** the two brace-bows (11) are interconnected with each other between the palate-support (15) and the pharyngeal wall support (16) via an elastic connecting element (P).

3. Mouth brace according to one of claims 1 or 2, **characterized in that** it comprises at least partially an elastically deformable wire (19).

4. Mouth brace according to claim 3, **characterized in that** this wire (19) is enclosed by a plastic sheath (10).

5. Mouth brace according to claim 4, **characterized in that** the plastic sheath (10) is soft and elastic.

6. Mouth brace according to claim 1, **characterized in that** it is composed of physiologically acceptable material.

7. Mouth brace according to claim 1, **characterized in that** it is manually or mechanically deformable.

8. Mouth brace according to claim 4, **characterized in that** it is provided with a plastic material being inserted after shaping between the wire (19) and the plastic sheath (10).

9. Mouth brace according to claim 8, **characterized in that** the plastic material being inserted after shaping is a silicone-like plastic.

10. Mouth brace according to claim 1 or 2, **characterized in that** it is integrally formed.

## Revendications

1. Appareil d'agrafe intra-oral (1) contre le ronflement, comprenant deux jambes d'agrafe (11), qui sont reliées l'une à l'autre par une pièce connecteur (12) élastique et à effet ressort, chacune des jambes d'agrafe (11) comprend une extrémité coudée (13), un arceau d'agrafe (14) et au moins un support du palais (15), et chaque jambe d'agrafe (11) est modelée et dimensionnée d'une manière qu'elle, lorsque insérée, vient, à sa côté du pharynx, avec le support du palais (15) en prise derrière la voile du palais d'un utilisateur (2), afin pour stabiliser ce palais, et à la côté de la lièvre, vient se buter avec l'extrémité coudée (13) contre la lèvre de l'utilisateur (2), de telle manière, que l'arceau d'agrafe (14) est serrée entre le palais mou et la lèvre de l'utilisateur (2),
**caractérisé en ce que**
la pièce connecteur (12) élastique et à effet ressort entre les deux jambes d'agrafe (11) comprend un support de la paroi pharyngée (16), qui se prolonge le long de la paroi pharyngée (5) jusque à la base de la langue (6) de la langue (7), et un support de base de la langue (17) situé au bout radical du support de la paroi pharyngée (16), c'est à la côté de la gorge, et qui sont modelées et dimensionnées de telle manière que, dans l'état inséré, le support de la parois pharyngée (16) vient se buter contre la paroi pharyngée (5) de l'utilisateur (2) et le support de la base de la langue (17) vient se buter contre la base de la langue (6) de l'utilisateur (2), afin pour stabiliser la paroi pharyngée (5) et la base de la langue (6) de l'utilisateur (2).

2. Appareil d'agrafe intra-oral selon la revendication 1, **caractérisé en ce que** les deux jambes d'agrafe (11) sont reliés par l'intermédiaire d'un élément élastique de liaison (P) entre le support du palais (15) et le support de la paroi pharyngée (16).

3. Appareil d'agrafe intra-oral selon l'une quelconque des revendications 1 ou 2, **caractérisé en ce qu'**il comprend au moins partiellement un fil (19) élastiquement modelable.

4. Appareil d'agrafe intra-oral selon la revendication 3, **caractérisé en ce que** le fil (19) est enrobé d'une gaine de matière plastique (10).

5. Appareil d'agrafe intra-oral selon la revendication 4, **caractérisé en ce que** la gaine de matière plastique (10) est souple et élastique.

6. Appareil d'agrafe intra-oral selon la revendication 1, **caractérisé en ce qu'**il se compose d'un matériau physiologiquement acceptable.

7. Appareil d'agrafe intra-oral selon la revendication 1, **caractérisé en ce qu'**il est manuellement ou mécaniquement déformable.

8. Appareil d'agrafe intra-oral selon la revendication 4, **caractérisé en ce qu'**il est pourvu entre le fil (19) et la gaine de matière plastique (10) d'une matière plastique insérée après le modelage.

9. Appareil d'agrafe intra-oral selon la revendication 8, **caractérisé en ce que** la matière plastique insérée après le modelage est une matière plastique de type silicone.

10. Appareil d'agrafe intra-oral selon une des revendications 1 ou 2, **caractérisé en ce qu'**il est formé d'une seule pièce.
